# EUROPEAN PATENT APPLICATION

(11) **EP 1 941 796 A2**
(43) Date of publication of application: **09.07.2008**
(21) Application number: 06819993.4
(22) Date of filing: 25.09.2006
(51) Int. Cl.: A01N 25/22, C07C 233/18

(54) **PESTICIDE FORMULATIONS WHICH RISK CRYSTALLISATION AND PROCESS FOR PRODUCING THE SAME**

(30) Priority: 26.09.2005 ES 200502416
(71) Applicant: Bayer CropScience Aktiengesellschaft, 42789 Monheim (DE)
(72) Inventor: CASAÑA GINER, Victor, A-Ebenfurth 2490 (AT); GIMENO SIERRA, Miguel, A-Ebenfurth 2490 (AT); GIMENO SIERRA, Barbara, A-2490 Ebenfurth (AT)
(74) Representative: Bader, Axel Jochen
(86) International application number: PCT/ES2006/000531
(87) International publication number: WO 2007/036585

(57) **Abstract**

Formulations are disclosed which contain pesticides which risk crystallising in water when the formulation which contains the pesticides as active ingredients is dissolved. The present formulations are characterised by long half-life ("shelf stability"), long crystallisation time once the formulation is dissolved in water, improved wettability and excellent emulsion stability.

## Description

### Subject of the invention

The subject of the present invention, as its title indicates, is represented by pesticide formulations that have the problem of crystallizing out in water on the dilution of the formulation containing them as active ingredients, and the formulations proposed here are characterized by a long half-life ("shelf stability"), a long crystallization time once the formulation has been diluted in water, an improved wettability and an excellent stability of the emulsion.
Another subject of the invention is a method for the preparation of these pesticide formulations.

The present invention relates to the field of pesticide formulations and anticrystallization mixtures.

### Prior art

**[Section 1]** One aspect of the invention is the improvement, vis-à-vis the prior art, of the stability of emulsifiable concentrates (known in the field as "ECs"), a term that due to its similarity to emulsions in water ("EW"), will be used in this invention for the two types of formulation), as well as of concentrated suspensions "CSs" and other types of formulations used in agriculture to combat parasites of all kinds.
**[Section 2]** For the industrial applicability of formulations with active ingredients (a.i.s) having a strong tendency to crystallize out (or whose crystals tend to increase in size) in water, it is necessary to keep the a.i. packaged (which the farmer will use for dilution with water before applying it in the field) without crystallization for a long time (e.g. for two years). Besides, once mixed with water, the a.i. should stay inside the oil droplets formed, without crystallizing out. Furthermore, when it comes to application in the field or in a place where a parasite is to be combated (e.g. lakes, water for its later use, containing vectors of diseases or bacteria, fungi, viruses, etc.), the filters, pre-filters and nozzles of the applicator devices get clogged up by the crystallized a.i., making the effective and uniform application of the a.i. impossible.
   Finally, the emulsions must comply with the FAO requirements, which state that the formulation should remain stable once mixed with water. This refers to the stability of the emulsion in the sense that it should not show a separation of the oil phase, or the cream/oil or the cream, once the formulation has been mixed with water. This stability does not only apply to the period immediately after the dilution of the formulation with water, but also to those frequent cases when the farmer has to leave the formulation that has already been diluted with water for a period that may be as long as several days (for example when the weather turns bad after the formulation has been diluted), especially when the product is to be applied over a large surface area.
**[Section 3]** In particular, pesticide formulations with a risk of crystallizing out when diluted with water because of their chemical structure and associated physicochemical properties can be improved, but the following phenomena can then easily occur when the product is emulsified or suspended in water:
   i) transfer from the oil phase to the aqueous phase in the case of ECs and EWs and precipitating out when placed in the water, owing to a low, but appreciable water solubility (between 10 mg/l and 400 mg/l of the "a.i." in water)
   ii) a rapid increase in the size of the crystals in the case of concentrated suspensions (CSs), microencapsulated types and other formulations in which crystals of the a.i. are already present (or can be present) in the formulation before it is diluted with water.
**[Section 4]** The closest prior art is exemplified by the fungicide tebuonazole, which possesses all the characteristics of easy crystallization in water and is an active ingredient that is the most difficult to formulate for later dilution of the formulation in water. Several formulations of this fungicide are covered by this patent. It should be mentioned in particular that there is currently no emulsifiable concentrate (EC) or emulsion in water (EW) on the market in the European Union (EU) that complies with the specification that the oil should not separate out 2 hours after emulsification in accordance with the international standard, CIPAC MT 36.1, accepted by the FAO. This shows the complexity of the formulation of tebuconazole and other products with a similar crystallization tendency.
**[Section 5]** ES2062597, which is equivalent to EP453899 by Bayer AG, describes N,N-dimethylalkylamides as anticrystallization agents and solvents in formulations of triazoles (a group of chemicals that includes tebuconazole). This solvent/anticrystallization agent is used commercially in the European Union in the product called Tebuconazole 250 EW (Folicur^{®}). ISP Investments claimed the use of alkyllactams as long ago as 1987 (EP311632) for use in agrochemical formulations, especially fungicides. This patent covered the use of N-octylpyrrolidone in concentrated emulsions. However, this patent was revoked: N-octylpyrrolidone had not been described earlier for agricultural use. In its EP391168, Bayer AG claimed the use of N-octylpyrrolidone for the third time in patent documents, this time for tebuconazole and/or triadimenol with an anticrystallization action. However, this patent does not relate to an emulsifiable concentrate as the present invention does, but to an aqueous liquid (a highly diluted aqueous mixture of the fungicide) that the farmer prepares just before application in the field. In the present patent, we describe the anticrystallizing (and not solvent) action of ethoxylated and/or propoxylated ethanolamides for use in emulsifiable concentrates themselves, this being the technical characteristic that is shared by all the claims and which makes a difference vis-à-vis the prior art. N,N-Dimethylalkylamides, like alkyllactams (especially N-octylpyrrolidone) simply have the property of being good solvents for triazole fungicides (in particular tebuconazole), and the fact that tebuconazole formulations containing these compounds avoid crystallization in a certain way is solely due to the fact that the active ingredient (tebuconazole) dissolves very well in the oil phase provided by these "incorrectly named" anticrystallization agents [as explained later in detail], so that tebuconazole does not go into the aqueous phase and does not crystallize out there. It may be said in a certain sense that water is the "anticrystallization agent" of the sugar sucrose, because it prevents its crystallization. However, no chemist would use the term "anticrystallization agent" for sugar to describe a property of water, but would simply say, correctly, that water is a solvent for sugar. The term "anticrystallization agent" is used in the present document appropriately, since the ethanolamides claimed are not good solvents for the compounds that form the subject of the present patent. The description and claims in EP391168 do not deal with an emulsifiable concentrate (as we do here), but with an aqueous mixture used by the farmer in his spraying tanks by the addition of N-alkyllactams (the idea to add N-octylpyrrolidone to an emulsifiable concentrate had not been patented by ISP Investments before or by other even earlier patents). In our invention, the presence of N-octylpyrrolidone has the sole function of providing an oil phase that dissolves the pesticide in question well and is not connected with the anticrystallization action described by Bayer in EP391168. Similarly, the present invention is not related at all to those inventions which describe phosphates (which are different from those mentioned here) with anticrystallization properties. Another fundamental difference from EP391168 is that the concentrates that can be emulsified in water (which are not claimed as such) that are described in the examples of EP391168 necessarily use water, while the emulsifiable concentrates in our invention do not call for the presence of water, although water can also be present. Furthermore, in our invention, the amount of the active ingredient (e.g. tebuconazole) can be even twice as much (25% wt/wt) as the amount disclosed in EP391168 (12.5%) (the risk of crystallization is greater at a higher concentration).
**[Section 6]** ES655197, which is equivalent to Bayer AG's EP655197, describes alkoxyalkyl phosphates with the suggestion of using them as anticrystallization agents in triazole formulations.
**[Section 7]** US3342673 (Mobil Oil Corp.) disclosed long before Bayer AG's EP453899 the use of N,N-dimethylalkylamides for use in emulsifiable concentrates. The only difference between this patent of Bayer AG and Mobil Oil Corporation's patent, which has already lapsed, is based on the mention, in US3342673, of their use for carbamates but not for triazoles (while EP453899 describes the use of N,N-dimethylalkylamides in emulsifiable concentrates for triazoles).
**[Section 8]** The products disclosed in EP453,899 and EP655197 have the disadvantage that they are hardly available on the market, are expensive and - most of all - do not ensure the required emulsion stability.

### Description of the invention

**[Section 9]** The present inventors have found certain mixtures that exert an anticrystallization effect which is similar to or even better than that disclosed in the documents mentioned above, and which at the same time confer an extraordinary emulsion stability on the ECs and EWs of tebuconazole and in general on pesticide formulations with similar crystallization problems and with a.i.s having similar water solubilities. The resulting emulsion stability is better than that of any product on the market on the basis of the standard international tests of CIPAC and FAO/WHO. In short, these formulations are better because they combine an excellent emulsification (stable emulsions, without the separation of a cream or oil) with the prevention of the formation of crystals. This problem has not been solved by the closest prior art, because its emulsions are poor (with the separation of oil or cream in a short space of time) or they exhibit partial crystallization. This problem is evident when the commercial formulations of certain pesticides, such as for example tebuconazole, are examined. The problem has now been solved by the inventors in the form set out below. It should be realized that the term "pesticide" as used in the present invention covers a single pesticide, its various isomeric forms, and a mixture of several pesticides.

### Brief description of the figures

A set of figures is included here to help to explain the invention. However, owing to the nature of the invention and the contents of the figures, it is best to leave their individual description until after the examples, which are given in the section headed "Preferred embodiment of the invention", since this makes for a better understanding and a more concise interpretation.

### Preferred embodiment of the invention

The present invention is based on a surprising property, never described before, of ethoxylated and/or propoxylated long-chain alkylethanolamides, which is that they dissolve and emulsify biocides with a tendency of crystallization and act as anticrystallization agents for them. Compounds of this type have been described in US4057506 as being useful for inclusion in heavy-duty detergents, but their use in agriculture is not mentioned there, nor of course are their anticrystallization properties. In particular, ethoxylated and/or propoxylated alkylmonoethanolamides are commercially described as "cleaning agents", and their use in agriculture is not known. They have never been described as crystallization inhibitors or solvents for ECs or EWs. An obvious extension of the invention lies in their use in concentrated suspensions, suspensions of capsules, powders, etc., where the abovementioned emulsifying and anticrystallization properties are utilized in the same way.

These ethanolamides have the general formula I: 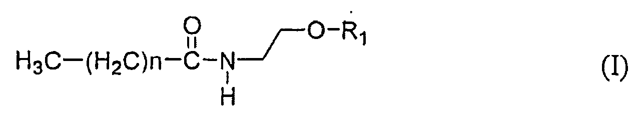 n = an integer that is not less than 4 and not more than 24
R₁ = an ethoxy group, repeated between 1 and 16 times, a propoxy group, repeated between 1 and 16 times, or an ethoxy-propoxy group, repeated between 1 and 8 times (with either a regular alternation or an arbitrary sequence of the ethoxy/propoxy groups).
The hydrocarbon chain can be either straight or branched.

It is an obvious extension, in view of the data presented in this invention, that the ethoxylated and/or propoxylated alcohol (monoethanol) group can be any short-chain alcohol or dialcohol, such as n-propanol, isopropanol, n-butanol, etc. Another obvious extension to the scope of the present invention is that the solvent and emulsifying effect may be fully retained even if more than 16 ethoxy (EO) and/or propoxy (PO) groups are used. However, beyond 16 mol of these, the solubilizing action (of the ethoxy and/or propoxy groups on the pesticide that tends to dissolve and then crystallize out in water) starts to be too high, which is inappropriate for the purposes of the present invention. The preferred amount is 2 to 8 mol of ethoxy and/or propoxy groups, but the suitable length can be ascertained by trial and error either within or outside these limits until the right number of moles is found for R₁ that gives a good emulsion for the pesticide or the mixture of chosen pesticides.

It is to be expected that the ethoxylated alkylethanolamide promotes the transfer of the active ingredient from the oil phase to the aqueous phase, thus promoting crystallization. It is well known to the expert in the field that, owing to the presence of ethoxy groups, an ethoxylated alkylethanolamide promotes the transfer of an a.i. emulsified in O/W to the aqueous phase more than the same ethanolamide that has not been ethoxylated does (provided that both of them solubilize the a.i.). It is generally obvious that, owing to the non-bonding electron pair of the oxygen atom, ethoxy or propoxy groups favor the solubilization of the active ingredient. However, it has now been found surprisingly by the inventors that these ethoxylated ethanolamines do not only solubilize the a.i. without incorporating it in the aqueous phase, but also inhibit its crystallization, which is the final effect that is hoped for and measured, and they ensure an extraordinarily advantageous emulsifying and wetting effect.

It is necessary to mention this difference vis-A-vis the prior art disclosed by EP453899. In the first place, apparently the compounds that are the most suitable for the dissolution and the prevention of the crystallization (preferred embodiment with the type of amide chosen and in fact the one used industrially in agrochemical formulations marketed under the trade name Hallcomid^{®} 8-10, Agnique^{®} KE 3308, Genagen^{®} 4296, Rhodiasolv^{®} ADMA 10, EP453899 are N,N-dimethyldecanamide and N,N-dimethyloctanamide: both compounds have one amide group and one alkyl chain with preferably 8 or 10 carbons.

The present invention makes use of ethoxylated and/or propoxylated alkylethanolamides that differ from the prior art disclosed by EP453899 in that:
i) To exert an effective action, the alkyl chain should have a greater length than the one disclosed, that is, preferably from 16 to 20 carbon atoms (double that in EP453899).
ii) Furthermore, EP453899 discloses N,N-dimethylalkylamides. In our case, there is no functional group with nitrogen atoms, which makes for a great difference in the chemical structure, polarity and several physicochemical properties between the two types of compounds.
iii) In addition, and this is a point of great importance as regards the chemical and functional differentiation vis-A-vis EP453899, in our invention, the alkylethanolamides are ethoxylated, which confers on them a completely different character from that described in EP453899. In EP453899, the N,N-dimethyldecanamides do not exert any emulsifying effect but are completely incorporated in the oil phase. However, the ethoxylated ethanolamides have an emulsifying effect, because the ethoxylated part tends to be located in the aqueous phase, while the alkylethanolamide part remains in the oil droplets. Surprisingly, the fact that the ethanolamides are ethoxylated and/or propoxylated does not mean that the readily crystallizing a.i. is incorporated in the aqueous phase and hence crystallizes out in a short time, as would be expected by the expert in the field. It is even more surprising that the use of the ethoxylated and/or propoxylated ethanolamide offers an extraordinarily advantageous effect for the ECs of easily crystallizing products: an impressive improvement in the stability of the emulsion. By tradition and in actual fact (e.g. in the case of Folicur) the emulsions of tebuconazole remain free of crystallization over a long time on the basis of a separation of the oil, occurring in only two to 6 hours (clearly, a poor emulsion, so that a high percentage of the a.i. is not found in the homogeneously emulsifiable liquid), while the use of ethoxylated alkylethanolamides prevents crystallization and also confers on the emulsion an extraordinary stability (no separation of the oil or cream in periods of more than two days).

In short, the use of ethoxylated ethanolamides (obviously in combination with other components of the formulations, since when used alone they cannot achieve all the necessary effects needed for an acceptable emulsion or suspension, which also applies to practically all co-formulants of any formulation) offers new, unexpected and advantageous properties for use in the formulation of the a.i. that readily crystallizes out in water.

The problem is that ethoxylated and/or propoxylated ethanolamides cannot act as sole co-formulants by themselves. To solve this problem, it is necessary to find an additional solvent, preferably one which also has anticrystallization properties and which acts jointly with alkylethanolamides in the desired way. The inventors have found that alkoxyalkyl phosphates, i.e. phosphoric acid esters with alkyl chains and ether groups in the said alkylol chain (different in chemical structure, physicochemical properties and in particular in solubilizing and emulsifying properties from the alkoxyalkyl phosphates without ether linkages, described in EP655197) have an extraordinary solubilizing effect - given their atypical chemical structure for a solvent for biocides with a low solubility in water. The use of an alkoxyalkyl phosphate (in combination with ethoxylated ethanolamides) is also presented here for the first time for application as an anticrystallizing and solubilizing agent at the same time.

These alkoxyalkyl phosphates have the general formula (II): 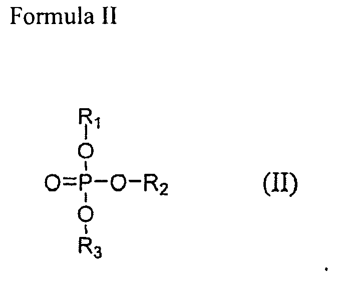 R₁ = -(CH₂)ₙ-O-(CH₂)ₘ or-H
R₂ = -(CH₂)ₓ-O-(CH₂)_{y} or-H
R₃ = -(CH₂)ᵤ-O-(CH₂)ᵥ or-H
n, x, u (independently of one another) = 1, 2, 3, 4, 5 or 6,
m, y, v (independently of one another) = 1, 2, 3, 4, 5, 6, 7, 8, 9 or 10
R₁, R₂ and R₃ are (independently of one anther):
i) either the above structure with the ether group
ii) or a hydrogen atom (but R₁, R₂ and R₃ cannot all be H at the same time).

For example, if no radical is a hydrogen atom, we have tris-(alkoxyalkyl) phosphates. If one radical is H, then the compound is a bis-(alkoxyalkyl) phosphate. The inventors would point out that the use of tris-(alkoxyalkyl) phosphates is preferred, these compounds having an ionic character. The presence of more ether linkages in the chains of R₁, R₂ and R₃ is an obvious extension of the compounds represented by (II), and they have similar characteristics as emulsifiers. Tris-(2-butoxyethyl) phosphate was described as long ago as 1929 in the patent DE523802 for a completely different use. Between then and now, no one has disclosed the use of (II) in the "tris" form (nor is it obvious) for use in agricultural formulations of compounds with a tendency to crystallize out in water on dilution.

As regards the use of an alkoxyalkyl phosphate with formula (II) according to the present invention, we have to point out an additional difference from the use of phosphates according to EP655197, in which patent the use of phosphates is given in the form of examples with anticrystallizing agents already disclosed by EP453899 or in combination with alkylbenzene sulphonates - something that does not occur in this invention.

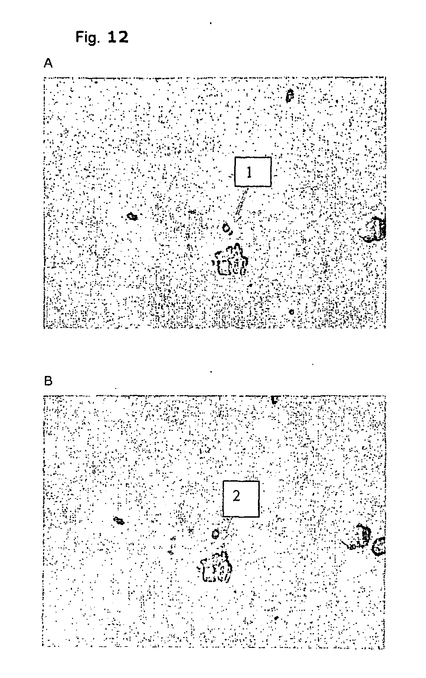

The problem is that the anticrystallization activity due to the previously described compounds belonging to this invention are sometimes not sufficiently effective under extreme conditions of use, that is, in regions where the ECs, EWs and CSs are kept at natural temperatures of below -15°C for a fairly long time, or where they stay in the applicator tanks for a long time (e.g. three weeks) after dilution. This problem of providing further resistance to crystallization that has already been improved is solved by the novel use, in ECs and EWs, of high-molecular hydrophilic polymers, which unexpectedly show anticrystallization properties (different from the well-known polypropylene glycols and polyethylene glycols). The inventors have surprisingly observed that the inclusion of polymers customarily employed as anticrystallization agents in concentrated suspensions is useful in emulsifiable concentrates, emulsions in water and microemulsions. These polymers are (but not exclusively) natural or synthetic polymers, such as polyvinylpyrrolidone, mixed polymers of polyvinylpyrrolidone with (meta)acrylates or polyvinyls, gum arabic, carboxymethylcellulose, polyvinyl alcohol, polyvinyl acetate, as well as derivatives and similar substances developed by specialized companies. The novelty lies in their new use in the context of ECs and EWs according to the present invention. The inventors have found that they exert an anticrystallization effect even before the crystal manages to form, as can be seen from Figs. 12A and 12B. For example, EP655197 shows that polymers of this type can be advantageously employed in sprayable liquids, but with the well-known adhesive activity - there is no mention of an anticrystallization action here. The use of polymers of this type in concentrated suspensions (CSs) is also part of the prior art, but with the difference that the CSs already contain crystals of an enormous size (unlike ECs and EWs, which should not contain any crystals of the a.i.), and the aim of these polymers is to prevent an excessive increase in crystal size. In other words, the prior art contains the use of these polymers as inhibitors of crystal growth but not as inhibitors that prevent the formation of crystals in oil phases. Therefore the prior art knows of the use of these polymers in agrochemical formulations in which the crystals are a constituent part of the same (e.g. wettable powders, granules that can be dispersed in water, and concentrated suspension), but their use in ECs for the prevention of crystallization does not belong to the prior art. The novelty of the introduction of these high-molecular polymers in the present invention lies in the utilization of their properties for forming a film outside the oil droplets (shown in Figs. 12A and 12B by a white halo around the oil droplets, which is absent if no hydrophilic polymer is added to the formulation), which film prevents any crystallization even before the smallest crystal manages to form. In the case when for some reason crystals have already formed, these crystals are prevented from "attracting" more active ingredient for their growth outside the oil droplets (Fig. 12A), nor is the agglomeration of crystals allowed. This can be seen from photomicrographs of the oil droplets in an emulsion according to the present invention and from comparative studies carried out with and without the said polymers. The prior art also knows of the use of these polymers in the applicator tank independently of the EC and EW; it is the farmer who introduces the polymers from a separate packaging unit from the one that contains the EC or EW - something that is not part of the invention.

The inclusion of a hydrophilic polymer of the type described in the previous paragraph in an oil-based formulation is also novel and required extensive studies. In particular, in the case of ECs and EWs not containing any water, the way of incorporating the polymer in the oil phase so that it dissolves in it is not obvious. Many tests carried out by the inventors show that if the co-formulants are not chosen suitably, the polymer is not solubilized, and unacceptable lumps are formed in the EC or EW. The problem is then how to incorporate a high-molecular polymer that inhibits the formation of crystals into an oil-based formulation (ECs contain an oil, the active ingredient and emulsifiers to permit the subsequent emulsification in water prior to the application in the field, but in the case of EWs or CSs, where water is already present before dilution takes place in the applicator tanks, it is easy to incorporate hydrophilic polymers (this is a difference vis-à-vis the prior art). The inventors have solved this problem by the use of a mixing process involving successive steps, as well as by the use of a novel mixture of co-formulants.

Furthermore, the distinctly apolar phase that should form oil droplets in which the active ingredient is to be dissolved and retained represents another problem to solve in compounds with a certain polarity, which are partially insoluble (for example those named in Section 24) in the majority of typical solvents in ECs and EWs (for example, light naphtha or heavy naphtha). This problem is difficult to solve and calls for a balance between the polarity of the apolar phase, the solubility of the active ingredient in it, and the partial solubility of the a.i. and the apolar phase in water, amongst other factors. For this purpose, the inventors have studied and chosen, as suitable co-formulants for this oil phase, certain compounds that are customarily used in agrochemical formulations, for example (but without any limitation being intended): N-octylpyrrolidinone, N-decylpyrrolidinone, N-dodecylpyrrolidinone (in general N-alkylpyrrolidinones with a C₁-C₁₆ alkyl group), gamma-butyrolactone, cyclohexanone, N-octylcaprolactam (in general N-alkylcaprolactams with a C₄-C₁₆ alkyl group), acetates and esters generally of fatty acids with a C₄-C₂₂ alkyl chain. as well as citrates, lactates, phthalates and oxalates. The inventors preferably opt for N-alkylpyrrolidinones, naphtha and gamma-butyrolactone, but not exclusively.

The compounds mentioned in the previous paragraph as possible substances to form oil droplets have the inconvenience that they have a certain solubility in water (except for naphtha), which constitutes a risk as regards the crystallization of the active ingredient, since they can act as phase transfer catalysts (from oil to water). For this reason, the inventors, to complete the solution of the problem of the formation of oil-based droplets, have found that alcohols, whether with a straight or branched chain, are surprisingly effective in creating robust oil droplets from which the active ingredient cannot leak out. Unlike many alcohols described in the literature for their use in ECs, and in particular in EP655197 (ES2117748, page 11, lines 60-62), the alcohols chosen in this invention are not short-chain alcohols (with fewer than 5 carbons). Nor does the invention cover the inclusion of alcohols, so they are only present in the formulations because they are used industrially in the preparation (solubilization) of sodium or calcium alkylaryl sulphonates, which belong to the prior art. In particular, straight-chain and branched alcohols, whether primary or tertiary, having preferably a C₅-C_{I2} chain are chosen as suitable compounds within the scope of this invention. The use of short-chain alcohols (with fewer than 5 carbons) has no advantage as regards the robustness of the apolar droplets under the conditions of the present invention. Beyond C₁₂, alcohols are not good solubilizers of the compounds covered by this invention.

In addition, suitable emulsifiers also have to be added in order to reduce the size of the droplets to prevent the separation of oil and cream. This prevention of a separation (improvement of the emulsion) is already ensured to a great extent by the use of ethoxylated and/or propoxylated ethanolamides; however, it is convenient to use emulsifiers, preferably - but not restrictively - from the group of polysorbates, ethoxylated and/or propoxylated fatty alcohols, ethoxylated and/or propoxylated castor oil, ethoxylated and/or propoxylated tristyrylphenols, as well as their mixtures and other commercial products made for this purpose.

Overall, the present invention relates to formulations that contain various compounds, each with its own function and interactions, which demonstrate their role when the EC and EW (in this case the concentrate already contains water) is diluted with water, ready for application:
i) main solvent for the active ingredient, consisting for example - but without limitation - of N-alkylpyrrolidones, N-alkylcaprolactams, naphtha, etc. (5-50% wt/wt)
ii) co-solvent for the active ingredient in the apolar droplets, preferably consisting of medium-chain (C₅-C₁₂) alcohols (2-25% wt/wt)
iii) co-solvent and anticrystallization agent for the active ingredient in the apolar droplets, which is partly located in the aqueous phase and consists of an alkoxyalkyl phosphate (10-80% wt/wt)
iv) emulsifier, anticrystallizing agent and co-solvent for the active ingredient, consisting of ethoxylated and/or propoxylated alkylethanolamides, whose ethoxy and/or propoxy end is located in the aqueous phase, while its hydrocarbon chain is located inside the oil drops (5-80% wt/wt)
v) distinctly emulsifying compounds located both in the aqueous phase and in the oil phase and belonging to the following groups (but not exclusively): polysorbates, ethoxylated and/or propoxylated fatty alcohols, ethoxylated and/or propoxylated castor oil, ethoxylated and/or propoxylated tristyrylphenols, and their mixtures (1-20% wt/wt)
vi) water-soluble polymers of the following types (but not exclusively): polyvinylpyrrolidone, mixed polymers of polyvinylpyrrolidone with (meta)acrylates or polyvinyls, gum arabic, carboxymethylcellulose, polyvinyl alcohol and polyvinyl acetate, as well as and derivatives and similar compounds developed by specialized companies, which polymers are located in the aqueous phase around the water droplets, whereby they prevent the approach of any crystal of the active ingredient that may have been formed in the aqueous phase and, above all, they inhibit the leakage of the active ingredient from the oil droplets into the aqueous phase (0.1-10% wt/wt).

The inventors have tried to determine the range and applicability of these formulations and give concrete list of pesticides. It is well known that different chemical compounds with different chemical structures need certain proportions and certain types of solvents, emulsifiers and other co-formulants for ECs and EWs. However, it is customary in the literature and in patents to extend a property (for example an anticrystallization or solvent ability) from one compound to another with a similar chemical structures.

This is what happened in the case of both EP655197 and EP453899, where the compositions for which the disclosed anticrystallization agents (N,N-dialkylamides) cover the whole range of triazoles with a fungicidal action, without giving examples of all of the compositions in which it is appropriate to use N,N-dimethylalkylamides. The inventors have found that some of the fungicides named in EP655197 and EP453899 do not have any crystallization problems at all. Propioconazole is an example. It has a chemical structure that is extraordinarily similar to that of tebuconazole, but is free of crystallization problems in an emulsifiable concentrate (EC) when one uses the conventional recipes recommended by the emulsifier manufacturers, or an emulsion in water (EW), provided that the EW contains less than 10% of water.

The inventors have established that it is not the similarly of the chemical structure that puts the pesticide "at risk" of crystallization in ECs and EWs, but instead it is much more sensible to evaluate certain experimental parameters (or parameters that are calculated semi-empirically) in order to predict the risk of crystallization. In numerous tests, the inventors have found some pesticides with a risk of crystallization and others without. This risk refers in concrete terms to what happens when 5 ml of the EC or EW is diluted with 95 ml of water and kept in a refrigerator (at 1°C), after which the emulsion is passed through a 5-µm filter 7 days later.

The abovementioned experimental parameters primarily refer to the preferential tendency of solubilization in water or octanol, known as the octanol/water partition coefficient, K_{o/w} or log P. The range of values of log P over which the invention is effective covers pesticides with a log P of between 2.5 and 4.5. Another value that affects the crystallization tendency is Henry's constant, which is connected with the electrical field inside the pesticide molecule and with the electrical field surrounding the oil droplets (molecules). Pesticides whose Henry's constant is less than 0.0003 Pa.m³/mol are covered by the present invention. This finding of the inventors is based on their own experience. Furthermore, the pesticides that come under the present invention are solid at ambient temperature, more specifically have a boiling point above 40°C. Finally, and this is very important, we have to consider the solubility of the pesticide in water. In the present invention, the water solubility range extends between 10 and 400 mg of pesticide per litre of water. This range means that at a very low solubility in water, the pesticide has a very low tendency to leave the oil droplets and crystallize out, while at solubilities exceeding 400 mg/l, the solubility starts to be sufficiently high to ensure that, if a good emulsifier system is chosen, the small amount of pesticide that enters the water remains in the dissolved form and does not crystallize out of the water. With these limitations, the preferred compositions according to this invention are not only not selected arbitrarily, but also those compositions are singled out here for which the invention is meaningful, instead of absurdly extending the scope of the invention on the basis of merely looking at the structure of the molecule.

With these selection criteria for the invention, the following pesticides can be listed, amongst many others, using their internationally accepted names:
atrazine, azinphos-methyl, bromadiolone, bromuconazole, butafenacil, chlorotoluron, coumatetralyl, cyclanilide, cyproconazole, 2,4-D, difenconazole, dimethomorph, diuron, ethoxysulfuron, fenamiphos, fenhexamid, ferimzone, flusilazole, fomesafen, fuberidazole, furalaxyl, halofenozide, imazalil, indanofan, iprovalicarb, isoproturon, linuron, MCPA, mefenpyr-diethyl, metconazole, methiocarb, nuarimol, paclobutrazole, propanil, prothioconazole, pyridaphenthion, siduron, simetryn, tebuconazole, triadimefon, triadimenol and triazamate. The subject of the invention also includes mixtures of any of the above pesticides with other pesticides or synergistic substances, since the favourable properties of the formulations discussed here do not necessarily imply that the oil droplets should contain either just one type of compound or more than one type. Moreover, it is possible to combine one of the above-mentioned pesticides with another one that is liquid at ambient temperature and which helps the inhibition of crystallization. Even other pesticides that are solid at ambient temperature will promote the inhibition of crystals of pesticides listed above when combined with those mentioned above that have a strong tendency to remain within the oil droplets. Since the combination of pesticides is so obvious as an extension of the present invention, it is not a subject of further discussion.

Obviously, the secondary subject is the formulation of an EC and EW of pesticides, which - thanks to the compounds and mixtures described here - can be formulated independently, whether they have crystallization problems or not. Although the main subject of the invention is represented by pesticides that readily crystallize out, as mentioned above, a formulation of any pesticide with the type of compounds described here is also included amongst those the inventors wish to be covered by in this invention, which is basically the correct formulation of pesticides in the EC and EW form. In the case where the pesticides are free of a risk of crystallization, the emulsifying and solvent properties of the ethoxylated and/or propoxylated alkylethanolamides and the alkoxyalkyl phosphate in combination (described for the first time in this invention) will obviously ensure a good emulsion, and are expressly included within the scope of this invention.

### Examples

Preferred embodiment of the invention (Example 1).

### Example 1

A series of tebuconazole formulations (1000 g) with the following compositions was prepared (percentages in terms of wt/wt):

| **Formulation:** | **A1** | **A2** | **A3** | **A4** | **A5** | **A6** | **A7** | **A8** |
|---|---|---|---|---|---|---|---|---|
| Tebuconazole | 20.6% | 20.6% | 20.6% | 20.6% | 20.6% | 20.6% | 20.6% | 20.6% |
| Hostaphat B310 | 20.0% | 15.0% | 5.0% | 20.0% | 40.0% | 25.0% | 18.0% | 5.0% |
| N-Octylpyrrolidinone | 20.0% | 3 5.0% | 27.4% | 12.0% | 10.0% | 20.0% | 18.0% | 32.0% |
| Octytdecanamide | 4 | | | | | | | |
| EO | 10.0% | 20.0% | 20.0% | 20.0% | 20.0% | 2.0% | 33.0% | 13.0% |
| Isooctanol | 20.0% | 2.0% | 18.0% | 5.0% | 5.0% | 20.0% | 6.0% | 12.4% |
| Tween 20 | 2.0% | 0.5% | 2.0% | 4.0% | 2.0% | 5.0% | 2.3% | 2.0% |
| Soprophor 796/P | 2.0% | 4.0% | 3.0% | 2.0% | 1.0% | 5.0% | 1.0% | 2.0% |
| Emulsogen EL 400 | 3.4% | 1.0% | 3.0% | 8.0% | 1.0% | 2.0% | 1.0% | 3.0% |
| PVDEM | 2.0% | 1.9% | 1.0% | 5.0% | 0.4% | 0.4% | 0.1% | 5.0% |
| Water | 0.0% | 0.0% | 0.0% | 3.4% | 0.0% | 0.0% | 0.0% | 5.0% |
| **Total:** | **100.0%** | **100.0%** | **100.0%** | **100.0%** | **100.0%** | **100.0%** | **100.0%** | **100.0%** |

To ensure perfect dissolution of the polyvinylpyrrolidone/methacrylate (PVDEM), a mixture was first prepared from the following ingredients (in the specified amounts): octyldecanamide 4 EO, isooctanol and N-octylpyrrolidone, to which the PVDEM was then added. Gentle heating and stirring gave a transparent solution, indicating that the PVDEM had fully dissolved - surprisingly - in a distinctly apolar phase. Another reactor was used at the same time to mix tebuconazole, Hostaphat B310, Soprophor 796/P, Tween 20 and Emulsogen EL 400. After mixing it with an anchor stirrer, this second mixture was obtained in a transparent form. The first mixture was finally incorporated in the second one, followed by mixing with an anchor stirrer until a fully homogeneous mixture was obtained.

The following tests were then carried out: CIPAC MT 36.1 (emulsification and re-emulsification), CIPAC MT 39.1 (stability in the cold) and CIPAC MT 46 (stability at elevated temperatures).

All the formulations were found to conform, with the tolerances established in the document "FAO Specifications for Plant Protection Products, Tebuconazole, AGP:CP/369", except for Formulation A6, which exhibited some crystallization, and Formulation A3, which showed an insufficient emulsification.

### Example 2

In this example, the stability of the commercial product Folicur^{®} Tebuconazole 250 EW was compared with that of a formulation similar to that described in Example 1, and specifically A1 [obtained by adding Soprophor^{®} 461 instead of Emulsogen^{®} EL 400, reducing the isooctanol to 10%, raising Hostaphat^{®} B310 to 26%, replacing Tween^{®} 20 by Tween^{®} 85, and replacing PVDEM by PVP-K15 (ISP)], which was characterized by HPLC-MS (Fig. 3) under the following analytical conditions: Agilent 1100 HPLC / SL ion trap, Hypersil ODS, mobile phase: acetonitrile, water and 0.1% of formic acid; AP-ESI detector, infrared analysis (Fig. 4), gas chromatography with flame ionization detector (Fig. 5A) and with mass spectrometer as the detector (Fig. 5B). This sample was called Formulation B.

The results of the emulsion stability test (CIPAC MT 36.1) were as follows:

| **Time after dilution** | **Parameter** | **Formulation B** | **Folicur^{®}** |
|---|---|---|---|
| 0 h | Original emulsion | Complete | Complete |
| 0.5 h | Cream | 0 ml | 0 ml |
| 2.0 h | Cream | 0 ml | 0 ml |
| | Oil | 0 ml | I ml |
| 24 h | Cream | 0 ml | 0.5 ml |
| | Oil | 0 ml | 3 ml |
| 24.0 h | Re-emulsification | Complete | Complete |
| 24.5 h | Cream | 0 ml | 0 ml |
| | Oil | 0 ml | 0 ml |

As can be clearly seen here, the emulsion stability of the Folicur^{®} sample was less good than that of Formulation B, for 1 ml of oil had already separated out after 2 hours in a 100-ml measuring cylinder containing the formulation at a concentration of 5%. The amount of oil separating out at the end of 24 hours was 3 ml. This period of time is normal under field conditions, when the applicator tanks are left half full, because the treatment has to be interrupted for bad weather or simply because of approaching night-time. Our Formulation B showed extraordinary emulsion properties. The oily residue separating out in the Folicur^{®} formulation was found to consist mainly of N,N-dimethyldecanamide.

A spraying test was also carried out at a pressure of 2 bar, using a 250-µm nozzle and a 150-µm filter of the type normally employed in the nozzles of applicator tanks. Two sprayable liquids were prepared - one based on Formulation B (0.25 litres in 100 litres of water) and the other based on Folicur^{®}. Both sprayable liquids were kept in 200-litre drums in a cold-room at 2°C for 4 days, after which the spraying test was conducted by recirculating 100 litres over 6 hours and spraying the liquid through a single nozzle. An extra drum was also used in which the sprayed liquid was collected for subsequent transfer back into the spraying tank, until the test was terminated after six hours of operation.

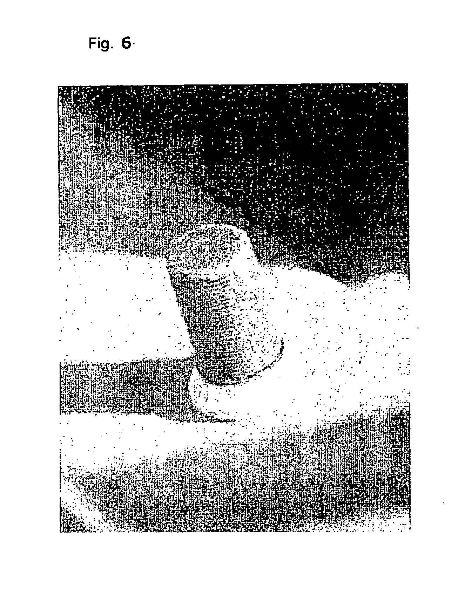

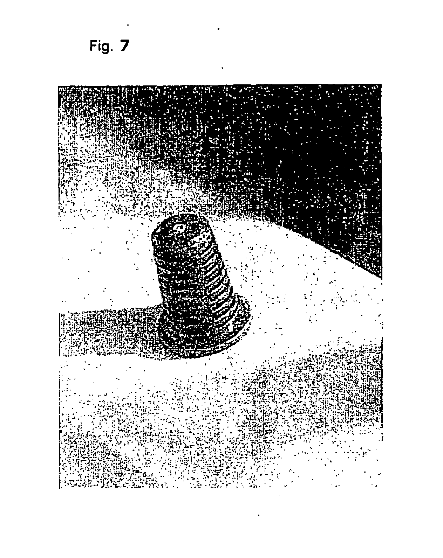

The results in Figs. 6 and 7 show that no blockage occurred either in the filters or in the nozzles with either of the two sprayable liquids (Formulation B and Folicur^{®}).

### Example 5

### Activity of a mixture of alkoxyalkyl phosphate and ethoxylated/propoxylated alkylethanolamines

In the EC composition described in Example 4, we replaced (in the same amounts) the Hostaphat B310 by the tri-(2-ethylhexyl) ester of phosphoric acid, the oleylethanolamide 4 EO by a mixture of equal parts of decanoic acid dimethylamide and octanoic acid dimethylamide, and called this Formulation C.

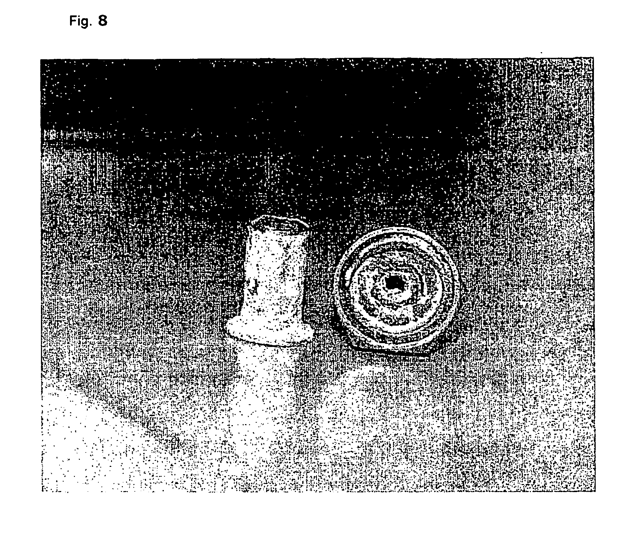

The same spraying test was carried out as in Example 4, at a pressure of 2 bar over 6 hours (100 litres with 250 ml of the emulsifiable concentrate "C"). The filters of the nozzles, and the nozzles themselves, were found to be blocked up after 12 minutes of operation, as shown in Fig. 8.

### Example 6

### Effect of the replacement of oxyalkane phosphates by alkyl esters according to the compositions described in ES21177843

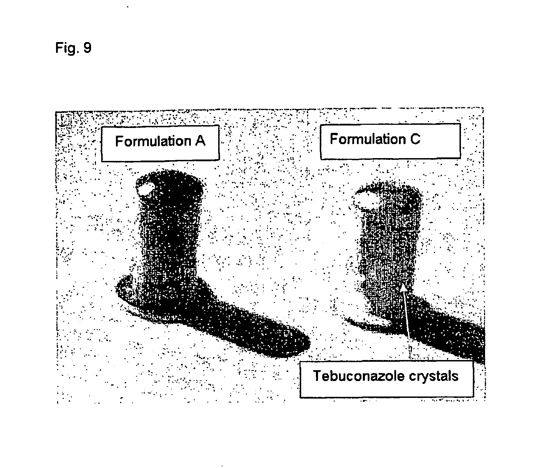

The procedure described in Example 5 was carried out in exactly the same way. In this case, Formulation B was modified by replacing Hostaphat^{®} B310 by the tributyryl ester of phosphoric acid. The spraying test was carried out as in Example 5, using a 150-µm metal filter with both this new Formulation E and with Formulation B. In the case of Formulation B, the filter was absolutely clean after six hours of operation, while the filter and the nozzle used in the test with Formulation E were clogged up, as shown in Fig. 9.

### Example 7

### Effect of the replacement of ethoxylated alkylethanolamines by N,N-dimethyldecanamides according to the compositions described in ES2062597

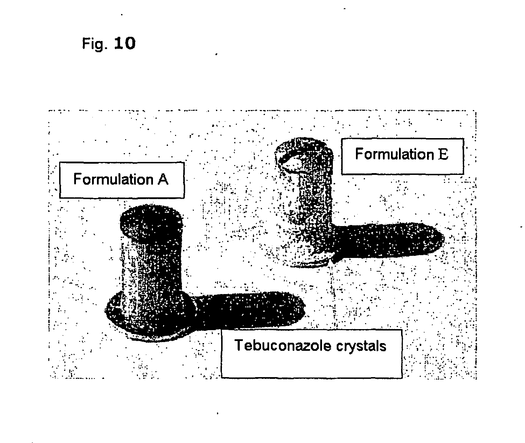

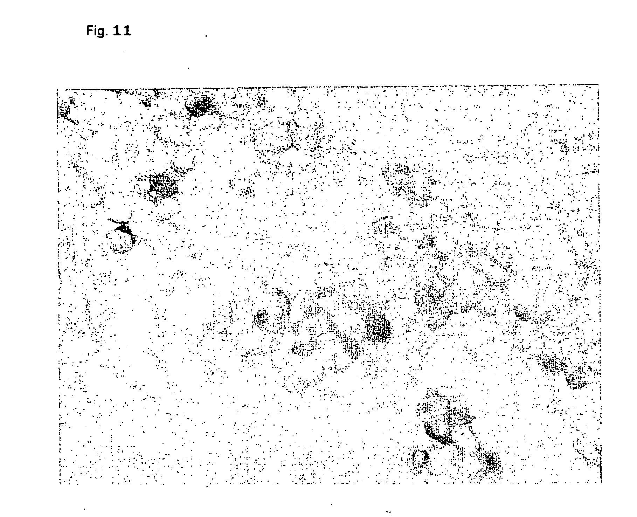

The test was carried out in exactly the same way as in Example 5. In this case, Formulation B was modified by replacing oleylethanolamide 4 EO by N,N-dimethyldecanamide. The spraying test was carried out as in Example 5, using a 75-µm metal filter with both this new Formulation F and with Formulation B. In the case of Formulation B, the filter was absolutely clean after six hours of operation, while the filter and the nozzle used in the test with Formulation E were clogged up, as shown in Figs. 10 and 11.

### Example 8

### Effect of the addition of PVP-K90 (ISP) on the crystallization stability

| **Formulation:** | **A1** | **A2** | **R1** |
|---|---|---|---|
| Tebuconazole | 20.6% | 20.6% | 20.6% |
| Hostaphat B310 | 0.0% | 0.0% | 20.0% |
| Dodecylpyrrolidone | 25.0% | 25.0% | 20.0% |
| Stearylethanolamide 6 EO | 25.0% | 25.0% | 10.0% |
| Tween 20 | 14.4% | 14.0% | 2.0% |
| n-Heptanol | 0.0% | 0.0% | 15.0% |
| Emulsogen EL 400 | 15.0% | 15.0% | 12.4% |
| PVP-K90 | | 0.4% | |
| **Total:** | **100.0%** | **100.0%** | **100.0%** |

### Test

1 g of each formulation was emulsified with 99 ml of water in 100-ml measuring cylinders. The emulsion was kept in a refrigerator at 4°C for 16 hours. It was finally filtered, and the crystals separating out were weighed. The value for the reference formulation was called 100, while the values for the other samples were expressed as a percentage of that for R1

| Results: | Mean ± SD* |
|---|---|
| A1 | 214.3 ± 25 |
| A2 | 169.2 ± 18 |

| | |
|---|---|
| * n = 3, deg. freedom = 5, P < 0.01 in Student's t test | |

The addition of PVP-K90 obviously improved significantly the inhibition of crystal formation in comparison with the results obtained for A1 and A2.

Figs. 12A and 12B show two oil droplets with a white halo, which is due to PVDEM. This shows that when the droplets come very close to one another (see A), the coalescence and/or possible rupture of the droplets are inhibited, and so is the crystallization. In photo B, taken seconds after photo A, we can see that not only the small droplet marked is separate from the other, larger one, but also that it does not adhere to or agglomerates with a crystal that was added in the test to check at the same time how hydrophilic polymers protect the droplets from the crystals formed.

### Example 9

### Inhibition of crystallization achieved by the inclusion of propoxylated alkylethanolamide in different types of pesticide, all having a Henry's constant of less than 0.0003 Pa.m³/mol, a boiling point of over 40°C, a water solubility of 10-400 mg/l and a log P value of 2.5-4.5

The pesticides described in Section 24 were used to prepare the following EW formulations. Since there was not enough capacity to carry out extensive tests with all the compounds on the list in Section 24, as well as for fine-tuning the amount of emulsifiers for a perfect emulsification, we restricted ourselves in this Example to the use the following basic formulation in all the cases (EO and PO indicate the number of moles of ethoxy and propoxy groups, respectively).

| **Formulation** | **Dn** |
|---|---|
| Pesticide | 5.0% |
| Hostaphat B310 | 23.0% |
| Gamma-Butyrolactone | 25.0% |
| Coco ethanolamide 3 PO | 25.0% |
| Tween 80 | 3.0% |
| n-Octanol | 8.0% |
| Tristyrylphenol 7 EO, 96 PO | 9.0% |
| PVP-K30 | 2.0% |
| **Total:** | **100.0%** |

The following table shows the qualitative results obtained in two different tests. In the crystallization test, an emulsion consisting of 2% of the formulation in 98 ml of water was kept at 1°C for 24 h and was then passed through a 25-µm metal filter. A minus sign indicates the absence of crystals, while a plus sign indicates their presence. The emulsion test shows the qualitative results obtained for the quality of the emulsion (phase separation). The worst emulsion, which however was still acceptable, scored +, a good emulsion scored ++ and the best ones scored +++. Both the phase separation and the time of its occurrence were observed when evaluating the emulsion. A triple plus sign (+++) means that no separation was noted at the end of 2 h, and complete re-emulsification was observed at the end of 24 h. A single + sign means that some oil did separate out after 2 hours.

| | **Crystallization** | **Emulsion** |
|---|---|---|
| atrazine | - | + |
| azinphos-methyl | - | +++ |
| bromadiolone | - | ++ |
| bromuconazole | - | +++ |
| butafenacil | - | ++ |
| chlorotoluron | - | +++ |
| coumatetralyl | + | ++ |
| cyclanilide | - | ++ |
| cyproconazole | - | ++ |
| 2,4-D | - | +++ |
| difenconazole | - | ++ |
| dimethomorph | - | ++ |
| diuron | - | +++ |
| ethoxysulfuron | - | ++ |
| fenamiphos | - | +++ |
| fenhexamid | - | ++ |
| ferimzone | - | + |
| flusilazole | - | ++ |
| fomesafen | - | +++ |
| fuberidazole, | - | ++ |
| furalaxyl | - | ++ |
| halofenozide | - | +++ |
| imazalil | - | ++ |
| indanofan | - | +++ |
| iprovalicarb | - | +++ |
| isoproturon | - | + |
| linuron | - | + |

| | **Crystallization** | **Emulsion** |
|---|---|---|
| MCPA | - | +++ |
| mefenpyr-diethyl | - | +++ |
| metconazole | - | ++ |
| methiocarb | - | +++ |
| nuarimol | - | ++ |
| paclobutrazole | - | +++ |
| propanil | - | +++ |
| prothioconazole | - | +++ |
| pyridaphenthion | - | ++ |
| siduron | + | ++ |
| simetryn | - | + |
| tebuconazole | - | ++ |
| triadimefon | - | ++ |
| triadimenol | - | ++ |
| triazamate | + | + |

As can be seen, only siduron and triazamate had crystallization problems, which indicates the "universality" of the mixtures listed in Section 19 for the inhibition of crystallization.

At the same time, the emulsions were not perfect in every case (in the sense of complying with the FAO requirements), but they can be improved by minor changes in the proportions of the emulsifiers and the N-alkylpyrrolidinones. In general, the majority of the emulsions are suitable for application in the field if used immediately after preparation. It is unlikely or even impossible that a group as large as that covered here will give an EC with good properties as regards the emulsion when one and the same basic formulation is used. However, with this basic formulation, the crystallization is indeed inhibited in almost every case, and the emulsions are close to the point where they only need a final adjustment based on trial and error, involving a reasonable number of tests (with about 25 tests at most, any of the abovementioned formulations can be improved to the point of becoming sufficiently acceptable).

### Example 10

### Irrelevant effect of the addition of water to the EC for the purpose of preparing an EW formulation

An aqueous emulsion formulation (EW) was prepared from the components described in Example 2, except that the amounts were reduced in equal proportions to make room for 4% of water, and the amount of the active ingredient was raised to 25.4%, making a difference between 25.4 and 20.6 in the isooctanol content in Formulation B. The results for the crystallization and the emulsification in Example 2 were exactly the same in this case. It follows from this that the incorrectly named "emulsions in water" (EW) (when the water content of the formulation is less than 10%) do not represent any complication as regards the advantageous effects of the formulations according to the present invention if one starts with an EC and then reduces the components in proportion in order to add the desired amount of water.

### Example 11

### Stability at an elevated temperature tested by the CIPAC MT 46 method

The formulation described in Example 5 was kept in a refrigerator at 1°C for three months in a heat-sealed 1-liter container made of polyterephthalate. Passing it through a 0.1-µm filter under vacuum after this period of time showed that the active ingredient had not crystallized out.

The figures attached here to help with the understanding of what has been said above are described below:

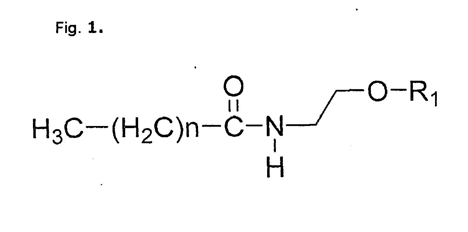

**Fig. 1** shows the compounds with Formula (I) where
   n = an integer that is not less than 4 and not more than 24
   R₁ = an ethoxy group, repeated between 1 and 16 times, a propoxy group, repeated between 1 and 16 times, or an ethoxy/propoxy group, repeated between 1 and 8 times (with either a regular alternation or an arbitrary sequence of ethoxy/propoxy groups). The hydrocarbon chain can be either straight or branched.

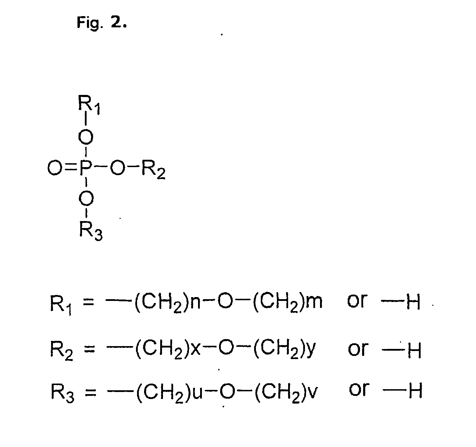

**Fig. 2** shows the compounds of Formula (II) 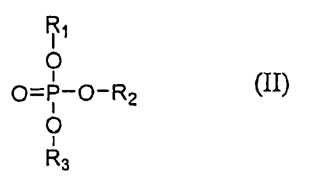 R₁ = -(CH₂)ₙ-O-(CH₂)ₘ or-H
   R₂ = -(CH₂)ₓ-O-(CH₂)_{y} or-H
   R₃ = -(CH₂)ᵤ-O-(CH₂)ᵥ or-H
   n, x, u (independently of one another) = 1, 2, 3, 4, 5 or 6,
   m, y, v (independently of one another) = 1, 2, 3, 4, 5, 6, 7, 8, 9 or 10
   R₁, R₂ and R₃ are (independently of one another):
   i) either the above structure with the ether group
   ii) or a hydrogen atom.

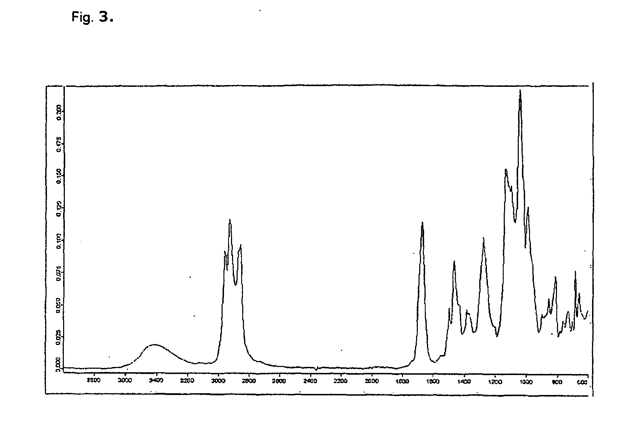

**Fig. 3** shows the FT-IR spectrum of a preferred embodiment of the invention, containing tebuconazole, isooctanol, ethoxylated octadecyldecanamide, 2-butoxyethyl phosphate, polyoxyethylene sorbitan trioleate, ethoxylated/propoxylated tristyrylphenol and polyvinylpyrrolidone.

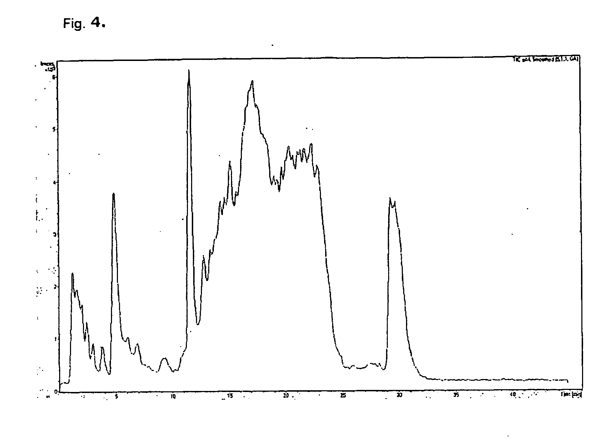

**Fig. 4** shows the chromatograph obtained by HPLC-MS for a preferred embodiment of the invention containing tebuconazole, isooctanol, ethoxylated octadecyldecanamide, 2-butoxyethyl phosphate, polyoxyethylene sorbitan trioleate, ethoxylated/propoxylated tristyrylphenol and polyvinylpyrrolidone

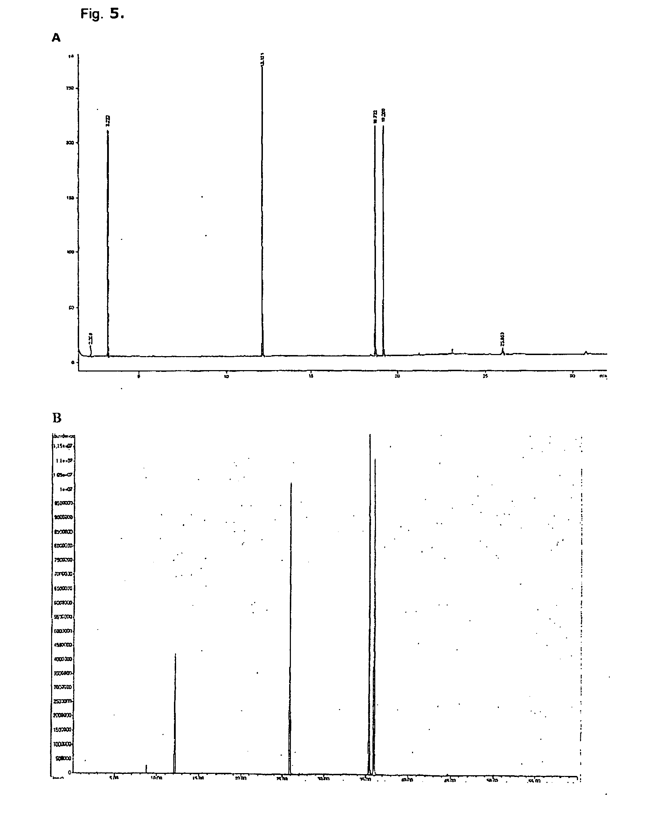

**Fig. 5**shows A) the chromatogram obtained by GC-FID for a preferred embodiment of the invention containing tebuconazole, isooctanol, ethoxylated octadecyldecanamide, 2-butoxyethyl phosphate, polyoxyethylene sorbitan trioleate, ethoxylated/propoxylated tristyrylphenol and polyvinylpyrrolidone (together with the fluoranene standard). MDM-12 column, 30 m x 0.25 µm x 0. 25 mm, flow rate: 2 ml/min, initial temperature: 50°C, final temperature: 290°C. B) chromatogram taken under the same analytical conditions, but using a mass spectrometer as the detector.
**Fig. 6** shows a filter after a spraying test with Formulation B
**Fig. 7** shows a filter after a spraying test with the Folicur® formulation
**Fig. 8** shows a filter and nozzle after a test with Formulation C
**Fig. 9** shows a filter after a test with Formulation E (on the right) and Formulation B (on the left)
**Fig. 10** shows a filter after a test with Formulation F (on the right) and Formulation B (on the left)
**Fig. 11** shows a photomicrograph of agglomerated crystals found in the filter illustrated in Fig. 9
**Fig. 12** shows photomicrographs illustrating the protective action of the hydrophilic polymer on the oil droplets dispersed in the aqueous phase. A crystal (the large object below the head of the arrow) was added to demonstrate also the crystallization-inhibiting effect on a crystal that had already formed.

## Claims

1. Pesticide formulations with a crystallization risk, essentially **characterized in that** ethoxylated and/or propoxylated alkylmonoethanolamides with Formula (I) are used 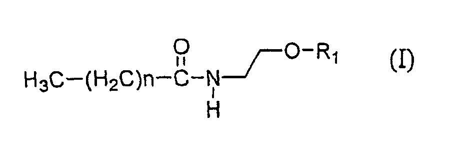 where
n = an integer that is not less than 4 and not more than 24
R₁ = an ethoxy group, repeated between 1 and 16 times, a propoxy group, repeated between 1 and 16 times, or an ethoxy/propoxy group, repeated between 1 and 8 times (with either a regular alternation or an arbitrary sequence of ethoxy/propoxy groups), and where the hydrocarbon chain shown on the left of the molecule can be either straight or branched, together with suitable co-formulants and one or more active substances, in the preparation of formulations of the type of emulsifiable concentrates and of the type of emulsions in water, with a view to inhibiting the crystallization of the active ingredients contained in the said formulations, both when the formulation is in concentrated form and when it is diluted with water for application in the field.

2. The pesticide formulations with a crystallization risk, as claimed in claim 1, **characterized in that** the value of n is preferably 13-21 and more preferably 16-20, and **in that** R₁ is an ethoxy group, repeated preferably 1-16 times and more preferably 2-8 times.

3. Pesticide formulations with a crystallization risk, essentially **characterized in that** compounds with Formula (II) are used: 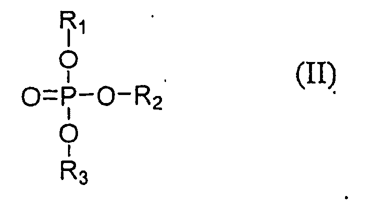 R₁ = -(CH₂)ₙ-O-(CH₂)ₘ
R₂ = -(CH₂)ₓ-O-(CH₂)_{y}
R₃ = -(CH₂)ᵤ-O-(CH₂)ᵥ
n, x, u (independently of one another) = 1, 2, 3, 4, 5 or 6,
m, y, v (independently of one another) = 1, 2, 3, 4, 5, 6, 7, 8, 9 or 10,
together with suitable co-formulants, including the compounds mentioned in claim 1, and one or more active substances, in the preparation of formulations of the type of emulsifiable concentrates and of the type of emulsions in water, with a view to inhibiting the crystallization of the active ingredients.

4. The pesticide formulations with a crystallization risk, as claimed in claim 3, **characterized in that** the phosphate of 2-butoxyethanol is used by preference.

5. Agrochemical compositions, **characterized in that** they contain:
i) main solvent for the active ingredient, chosen from a group comprising N-alkylpyrrolidones, N-alkylcaprolactams, naphtha, cyclohexanone and gamma-butyrolactone, in an amount of 5-40% (wt/wt)
ii) co-solvent, chosen from amongst straight-chain or branched alcohols with a C₅-C₁₂ chain, in an amount of 2-25% (wt/wt)
iii) co-solvent and anticrystallization agent, chosen from a group comprising the compounds mentioned in claim 3, in an amount of 10-80% (wt/wt)
iv) emulsifier, anticrystallization agent and co-solvent for the active ingredient, chosen from a group comprising the compounds mentioned in claim 1, in an amount of 5-80% (wt/wt)
v) distinctly emulsifying compounds, chosen from a group comprising polysorbates, ethoxylated and/or propoxylated fatty alcohols, ethoxylated and/or propoxylated castor oil, ethoxylated and/or propoxylated tristyrylphenols, and their mixtures, in an amount of 1-20% (wt/wt)
vi) water-soluble polymers of the type of polyvinylpyrrolidone, mixed polymers of polyvinylpyrrolidone with (meta)acrylates or polyvinyls, gum arabic, carboxymethylcellulose, polyvinyl alcohol and polyvinyl acetate, as well as derivatives and copolymers of the same, in an amount of 0.1-10% (wt/wt)
vii) optionally water in an amount of 0.5-60% (wt/wt).

6. Pesticide formulations with a crystallization risk, according to the compounds described in claims 1 and 3, **characterized in that** the formulations of the active agrochemical ingredients used are **characterized by** having:
i) a log P of between 2.5 and 4.5
ii) a Henry's constant of less than 0.0003 Pa.m³/mol
iii) a boiling point of over 40°C at atmospheric pressure
iv) a water solubility of 10-400 mg/l at 20°C.

7. Pesticide formulations with a crystallization risk, according to the compounds described in claims 1 and 3, **characterized in that** the compounds used in the formulations of the agrochemical active ingredients are chosen from the group of: atrazine, azinphos-methyl, bromadiolone, bromuconazole, butafenacil, chlorotoluron, coumatetralyl, cyclanilide, cyproconazole, 2,4-D, difenconazole, dimethomorph, diuron, ethoxysulfuron, fenamiphos, fenhexamid, ferimzone, flusilazole, fomesafen, fuberidazole, furalaxyl, halofenozide, imazalil, indanofan, iprovalicarb, isoproturon, linuron, MCPA, mefenpyr-diethyl, metconazole, methiocarb, nuarimol, paclobutrazole, propanil, prothioconazole, pyridaphenthion, siduron, simetryn, tebuconazole, triadimefon, triadimenol and triazamate.

8. Pesticide formulations with a crystallization risk, which include polyvinylpyrrolidone and/or polyvinyl alcohol, **characterized in that** the polyvinylpyrrolidone and/or polyvinyl alcohol are used in combination with the compounds described in the preceding claims.

9. An agrochemical formulation in the form of an emulsifiable concentrate, **characterized in that** it has: an infrared spectrum like that shown in Fig. 3, a chromatogram shown in Fig. 4, obtained by HPLC / mass spectrometry under the analytical conditions mentioned in the Description, a gas-chromatogram shown in Fig. 5A obtained with a flame ionization detector, a gas-chromatogram shown in Fig. 5B, obtained with a mass spectrometer used as a detector, and **in that** it contains - as its ingredients - tebuconazole, isooctanol, ethoxylated octadecyldecanamide, 2-butoxyethyl phosphate, polyoxyethylene sorbitan trioleate, ethoxylated/propoxylated tristyrylphenol and polyvinylpyrrolidone.

10. The agrochemical formulation in the form of an emulsion in water as claimed in claim 5, **characterized in that** it is composed of tebuconazole, isooctanol, ethoxylated octadecyldecanamide, 2-butoxyethyl phosphate, polyoxyethylene sorbitan trioleate, ethoxylated/propoxylated tristyrylphenol, polyvinylpyrrolidone and water.

11. A method for formulating EC and EW, containing hydrophilic polymers as claimed in claim 5 v), **characterized in that**:
a) the polymer as claimed in claim 5 v) is dissolved with the compounds as claimed in claim 5 i), ii), iii) and iv)
b) the a.i. is dissolved with the compounds as claimed in claim 5 v) and iii)
c) a) and b) are mixed together
d) the hydrophilic polymer according to 5 v) is added to mixture c)
e) water is added optionally for an emulsion in water
f) the final mixture is homogenized
g) it is optionally filtered,
heating being optional in any of the above stages.
